(19) Europäisches Patentamt
European Patent Office
Office européen des brevets

(11) Publication number: **0 117 647**
**B1**

(12) # EUROPEAN PATENT SPECIFICATION

(45) Date of publication of patent specification: **17.08.88**

(51) Int. Cl.$^4$: **C 07 C 91/34,** C 07 C 103/29, C 07 C 147/12, A 23 K 1/16

(21) Application number: **84300559.6**

(22) Date of filing: **30.01.84**

(54) **Improvements in or relating to phenethanolamines.**

(30) Priority: **31.01.83 US 462587**

(43) Date of publication of application:
**05.09.84 Bulletin 84/36**

(45) Publication of the grant of the patent:
**17.08.88 Bulletin 88/33**

(84) Designated Contracting States:
**BE CH DE FR GB IT LI LU NL SE**

(56) References cited:
**EP-A-0 057 900**
**EP-A-0 089 154**
**DE-C- 894 396**
**FR-A-2 430 407**
**FR-A-2 430 409**
**FR-M- 6 255**
**US-A-4 391 826**

**RECUEIL DES TRAVAUX CHIMIQUES DES PAYS/BAS, vol. 92, 1973. J. VAN DIJK, H.D. MOED: "Synthesis of B-phenylethylamine derivatives X; n-(hydroxy- and methoxy-aralkyl) derivatives", pages 1281-1297**

(73) Proprietor: **ELI LILLY AND COMPANY**
**307, East McCarty Street**
**Indianapolis Indiana 46285 (US)**

(72) Inventor: **Anderson, David Bennett**
**1334 Greenhills Road**
**Greenfield Indiana 46140 (US)**
Inventor: **Schmiegel, Klaus Kurt**
**4507 Staughton Drive**
**Indianapolis Indiana 46226 (US)**
Inventor: **Veenhuizen, Edward Lee**
**R.R. 3, Box 41**
**Greenfield Indiana 46140 (US)**
Inventor: **Tuttle, Ronald Ralph**
**2704 Vista Del Sembrado**
**Escondido, California 92025 (US)**

(74) Representative: **Hudson, Christopher Mark et al**
**European Patent Attorney Erl Wood Manor**
**Windlesham Surrey GU20 6PH (GB)**

**EP 0 117 647 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The chemistry and use of β-phenethanolamines has been extensively investigated. A number of these compounds have been reported to have beneficial cardiac activities; see U.S. Patent No. 3,987,200. Such compounds also are known to have sympathomimetic activity, and have found utility as utero-relaxing agents; Van Dijk et al., *Recueil, 92,* 1281 (1973). More recently, a group of β-phenethanolamines have been reported as possessing anti-hyperglycemic activity, and have been found effective in promoting the loss of weight in animals, U.S. Patent No. 4,391,826. Further β-phenethanolamines are disclosed in French Medicament Patent No. 6.255, West German Patent No. 894396 and French Patent No. 2.430.407.

This invention provides a method for increasing weight gain in domestic animals and improving the efficiency of feed utilization and the quality of the carcass. The invention is more particularly directed to a method for promoting growth and improving feed efficiency and leanness comprising administering a compound having the formula (I)

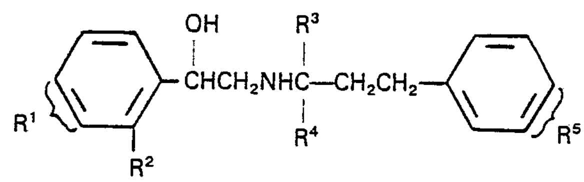

(I)

wherein:

$R^1$ is hydrogen, hydroxy, or methoxy;

$R^2$ is hydrogen or fluoro,

$R^3$ is hydrogen or $C_1$—$C_2$ alkyl;

$R^4$ is methyl;

$R^5$ is hydrogen, fluoro, nitro, hydroxy or $SO_2CH_3$; provided that $R^1$ is hydrogen only when $R^5$ is nitro or $SO_2CH_3$ and provided that when $R^1$ and $R^5$ are both *para* hydroxy and $R^2$ is hydrogen, $R^3$ is $C_1$—$C_2$ alkyl; and the acid addition salts thereof.

A preferred method for promoting growth and improving feed efficiency and leanness according to this invention employs a compound of the above formula wherein $R^1$ is hydroxy, $R^2$ is hydrogen, $R^3$ is hydrogen or methyl and $R^4$ is methyl. The method is most preferably practiced employing a compound wherein $R^1$ and $R^5$ both are hydroxy and $R^2$ and $R^3$ both are hydrogen and $R^4$ is methyl. When $R^1$ is hydroxy or methoxy, it preferably is in the *para* position. When $R^5$ is other than hydrogen, it also is preferably in the *para* position.

This invention also provides an animal feed premix comprising a β-phenethanolamine of the above formula together with a suitable carrier therefor.

The compounds are either known in the art or can be readily prepared by well known synthetic procedures. A preferred compound is 1 - (3 - hydroxyphenyl) - 2 - [1 - methyl - 3 - (4 - hydroxyphenyl)-propylamino]ethanol. This β-phenethanolamine is disclosed in South African Patent No. 673,994 published in May, 1967. A related compound, 1 - (4 - hydroxyphenyl) - 2 - [1 - methyl - 3 - (4 - hydroxyphenyl)-propylamino]ethanol, is disclosed as having utero-relaxing activity by Van Dijk et al. in *Recueil, 92* 1281 (1973), and is the subject of our divisional patent application, European Patent Application No. 86106094.5.

The compounds of formula (I) can be prepared by reaction of a styrene oxide with a 3-phenylpropylamine derivative. For example, a styrene oxide such as 2-fluorostyrene oxide can be reacted with about an equimolar quantity of an amine such as 1-methyl-3-(4-nitrophenyl)propylamine in an unreactive organic solvent such as ethanol, methanol, *n*-propanol, dioxane, or the like. The reaction generally is carried out at a temperature of about 50 to about 120°C., and at such temperature the reaction routinely is substantially complete within about 6 to about 10 hours. The product, a β-phenethanolamine, is readily isolated by simply removing the reaction solvent, for instance by evaporation under reduced pressure, and further purification can be accomplished if desired by standard techniques, including crystallization, chromatography, acid-base extraction, and the like.

An alternative method for preparing the β-phenethanolamines comprises reacting a mandelic acid derivative with a 3-phenylpropylamine derivative to provide an amide, which upon subsequent reduction provides a compound of the above formula. For example, a phenylpropylamine derivative such as 1-methyl-3-(3-fluorophenyl)propylamine can be reacted with an acylating agent such as a hydroxy protected mandelic acid halide, or preferably simply reacted with a mandelic acid in the presence of a common peptide coupling reagent such as N,N'-dicyclohexylcarbodiimide, carbonyldiimidazole, N-ethoxycarbonyl-2-ethoxy-1,2-dihydroquinoline, commonly referred to as EEDQ. The direct coupling reaction generally is conducted in an organic solvent such as benzene or N,N-dimethylformamide, and normally is complete after about 2 to 48 hours when carried out at about −30 to about 100°C. The product is an amide that is readily isolated by simply filtering the reaction mixture and then removing the reaction solvent. The amide thus formed is next reduced by reaction with a common reducing agent such as diborane or the like to provide a β-phenethanolamine defined by the above formula.

A similar, yet alternative, method of synthesis comprises reacting a phenethanolamine with a phenylethyl ketone to provide a Schiff base, which upon reduction gives a compound to be employed in the present method.

It should be noted that the compounds to be employed in this invention possess at least one asymmetric center (i.e. the carbinol center), and when $R^3$ and $R^4$ differ, the compounds possess two asymmetric centers. Since employment of individual optical isomers necessitates preparing the β-phenethanolamines from optically active starting materials, or using costly separation procedures, a preferred embodiment of this invention for veterinary purposes employs a mixture of optical isomers. The mixture of optical isomers is employed in the method without subsequent separation of isomers.

Since the β-phenethanolamines to be employed in the present method are inherently basic, they readily form acid addition salts with any number of inorganic and organic acids. These salts can be employed in the invention, and often are preferred to the free base since they generally are more soluble in solvents such as water and are more conveniently formulated as an animal feedstuff. Acids commonly employed to form acid addition salts include mineral acids such as hydrochloric acid, sulfuric acid, phosphoric acid, perchloric acid and the like; and organic acids such as acetic acid, citric acid, succinic acid, para-toluene sulfonic acid, methanesulfonic acid, lactic acid and the like. Preferred salts to be employed in the present method include the hydrochlorides and hydrobromides.

Typical β-phenethanolamines to be employed in the method of this invention include the following:

1-(2-fluoro-4-hydroxyphenyl)-2-[1-methyl-3-(4-fluorophenyl)propylamino)ethanol;

1-(4-methoxyphenyl)-2-[1-methyl-3-(4-hydroxyphenyl)propylamino]ethanol;

1-phenyl-2-[1,1-dimethyl-3-(3-methylsulfonylphenyl)propylamino]ethanol;

1-(4-hydroxyphenyl)-2-[1,1-dimethyl-3-(4-hydroxyphenyl)propylamino]ethanol hydrochloride;

1-(phenyl)-2-[1-methyl-3-(4-nitrophenyl)propylamino]ethanol;

1-(3-hydroxyphenyl)-2-[1-methyl-3-(4-fluorophenyl)propylamino]ethanol succinate;

1-(4-hydroxyphenyl)-2-[1,1-dimethyl-3-phenylpropylamino]ethanol hydrobromide; and

d-1-(4-hydroxyphenyl)-2-[1,1-dimethyl-3-(4-hydroxyphenyl)propylamino]ethanol.

The method of promoting growth and improving leanness and feed efficiency provided by this invention is practised by administering an effective amount of a compound of formula (I) to a domesticated animal that receives a nutritionally adequate diet. The term "domesticated animal" as used herein includes those warm-blooded animals raised for human meat consumption, for example grower/finisher swine, poultry, ruminants and the like, but excludes such animals as rats. In a preferred embodiment, the growth of pigs, chickens and turkeys is promoted employing a β-phenethanolamine. Another preferred embodiment is practiced in ruminants such as cattle, sheep and goats. The method of improving leanness is not limited to meat producing animals, and can be practiced on other warm-blooded animals, including dogs and cats. This latter embodiment is particularly useful when it is desired to maintain mature animals in a relatively lean physical state.

The method of the invention is preferably practiced by orally administering an effective amount of a β-phenethanolamine to an animal. Other routes of administration can be employed, for instance intramuscular or intravenious injection; however, such routes are less practical. The amount to be administered to an animal is the amount that is effective in causing a promotion of growth, or an improvement in the efficiency of utilization of food, or an improvement in carcass quality of the animal, for instance in the form of less fatty tissue and improved leanness. The effective amount to be administered will vary somewhat depending upon the particular animal species being treated and the particular active ingredient employed, but generally will be from about 1 to about 1000 parts per million (ppm) of total daily feed intake. Such amount will provide a dosage of about 0.05 to about 50 mg/kg. A preferred embodiment employs about 1 to about 200 ppm, and more preferably from about 5 to about 100 ppm. A typical amount of active ingredient to be administered to swine will be from about 5 to about 40 ppm. For example, when practicing the method in animals such as ruminants or swine, the compound will be added to the daily feed ration of about 5 to about 100 parts per million of the daily feed ration.

For oral administration, the active β-phenethanolamine is preferably admixed with suitable carriers or diluents commonly employed in animal husbandry. Animal feed premixes comprising a β-phenethanolamine as defined herein are provided as a further embodiment of this invention. Typical carriers and diluents commonly employed in such feedstuffs include corn meal, soybean meal, alfalfa meal, rice hulls, soybean mill run, cottonseed oil meal, bone meal, ground corn, corncob meal, sodium chloride, urea, cane molasses and the like. Such carriers promote a uniform distribution of the active ingredient in the finished feed ration into which such compositions are added, thereby ensuring proper distribution of the active ingredient throughout the feed. The feedstuff composition provided by the invention will contain about 5 to about 95 percent by weight of active ingredient, and more typically about 10 to about 50 percent by weight. As already noted, the acid addition salts of the active phenethanolamines are generally preferred for oral administration, and are thus the preferred form of active ingredient for the feedstuff compositions of the invention.

While the preferred method for orally administering the growth promoters is via the daily feed rations, the compounds can be incorporated into salt blocks and mineral licks, as well as being added directly to drinking water for convenient oral consumption. The compounds can additionally be formulated with polymorphous materials, waxes and the like for long-term controlled release, and administered to an animal as a bolus or tablet only as needed to maintain the desired daily payout of active ingredient.

For parenteral administration, the β-phenethanolamines can be admixed with conventional carriers such as corn oil, sesame oil, carbowax, calcium stearate and the like. Such formulations can be molded into pellets and administered as an injection or as a slow-release subcutaneous implant. Such administrations can be made as often as needed to ensure the proper dosing of active ingredient to obtain the desired rate of growth promotion and improvement in leanness and feed efficiency.

While the compounds described herein are effective in promoting average daily weight gain and improving feed efficiency in animals, they also cause observable improvement in the quality of the meat produced. For example, the compounds appear to mobilize free fatty acids from fatty tissue and depress the deposition of fat as the animals gain weight. This reduction of fat is beneficial since the animal being treated according to the invention gains weight in the form of more useable lean meat, thereby reducing waste and improving the value of the animal thus treated. Also, mature animals that are not subject to additional weight gain can be maintained in a desirably lean form by administering a compound as described herein.

The practice of the present invention is more fully illustrated by the following detailed examples.

Example 1

Preparation of dl-4-(benzyloxy)mandelic acid

A solution of 5.0 g of dl-4-hydroxy mandelic acid, 11.0 g of benzyl chloride and 10.0 g of potassium carbonate in 50 ml of methanol was heated to reflux and stirred for seventy-two hours. The reaction mixture was cooled to room temperature and diluted with 50 ml of water. The aqueous solution was washed twice with 50 ml portions of benzene, and then was acidified with concentrated hydrochloric acid. The aqueous acid solution was extracted three times with 50 ml portions of ethyl acetate. The organic extracts were combined, washed with water and with saturated sodium chloride solution and dried. Removal of the solvent by evaporation under reduced pressure provided 5.7 g of a solid which was then recrystallized from 300 ml of toluene to afford 5.33 g of dl-4-(benzyloxy)mandelic acid. M.P. 153—155°C.

Analysis calc. for $C_{15}H_{14}O_4$

Theory: C, 69.76; H, 5.46;
Found: C, 69.96; H, 5.33.

Example 2

Resolution of dl-4-(benzyloxy)mandelic acid to provide R(−)-(4-benzyloxy)mandelic acid

To a stirred solution of 185.6 g of dl-4-(benzyloxy)mandelic acid in 2500 ml of ethyl acetate at 80°C was added in one portion 43.6 g of R(+)-α-methylbenzylamine. The reaction mixture was cooled to room temperature, and the precipitated solid which had formed was collected by filtration and washed with fresh ethyl acetate. The solid was recrystallized twice from a solution of ninety percent ethanol in water to provide 91.4 g of the R(+)-α-methylbenzylamine salt of R(−)-4-(benzyloxy)mandelic acid. M.P. 208.5—209.5°C. $[\alpha]_D$ −38.6°, $[\alpha]_{365}$ −155.3° (MeOH)

Analysis calc. for $C_{23}H_{25}NO_4$

Theory: C, 72.80; H, 6.64; N, 3.69;
Found: C, 72,95; H, 6.83; N, 3.95.

To a stirred suspension of 91.4 g of the above-named salt in 2000 ml of ethyl acetate was added 150 ml of ten per cent aqueous hydrochloric acid solution. The aqueous acid solution was separated, and the organic layer washed with water and dried. Removal of the solvent by evaporation under reduced pressure afforded 54.5 g of R(−)-4-(benzyloxy)mandelic acid, ie. R(−)-2-(4-benzyloxyphenyl)-2-hydroxyacetic acid. M.P. 155—161°C $[\alpha]_D$ −102.2°; $[\alpha]_{365}$ −410.6° (MeOH)

Analysis calc. for $C_{15}H_{14}O_4$

Theory: C, 69.76; H, 5.46;
Found: C, 69.67; H, 5.41.

Example 3

Preparation of dl-1-methyl-3-(4-benzyloxyphenyl)propylamine

A solution of 40.0 g of methyl 2-(4-benzyloxyphenyl)ethyl ketone and 160 ml of anhydrous ammonia in 300 ml of ethanol was heated at 75°C and stirred for two hours. After cooling the reaction mixture to room temperature, 4.0 g of Raney nickel was added in one portion, and the reaction mixture then was stirred at 25°C for twelve hours under a hydrogen atmosphere at 300 psi. The reaction mixture next was filtered and the filtrate was concentrated by evaporation of the solvent under reduced pressure to provide an oil. The oil was dissolved in 120 ml of 3N hydrochloric acid, from which the product as a hydrochloride salt precipitated. The salt so formed was collected by filtration and recrystallized from methanol and toluene to provide 36.2 g of dl-1-methyl-3-(4-benzyloxyphenyl)propylaminium chloride. M.P. 195—197.5°C.

Example 4

Resolution of dl-1-methyl-3-(4-benzyloxyphenyl)propylamine to provide R-(−)-1-methyl-3-(4-benzyloxyphenyl)propylamine

A solution of 629.3 g of dl-1-methyl-3-(4-benzyloxyphenyl)propylaminium chloride was converted to the free amine by reaction with 95 g of sodium hydroxide in 400 ml of water. The free amine was then

dissolved in 2000 ml of methylene chloride and added to a solution of 328 g of D-(−)-mandelic acid in 1000 ml of methanol. The mandelic acid salt of the free amine precipitated out of solution and was recrystallized three times from methanol to provide 322 g of the R-mandelic acid salt of R-1-methyl-3-(4-benzyloxyphenyl)propylamine. M.P. 166—167°C. $[\alpha]_D$ −30°, $[\alpha]_{365}$ −119° (MeOH).

The salt so formed was converted to R-1-methyl-3-(4-benzyloxyphenyl)propylamine as the free base by reaction with aqueous sodium hydroxide.

Example 5A

1-Phenyl-2-[1-methyl-3-(4-nitrophenyl)propylamino]ethanol, hydrochloride

A solution of 5.0 g (25.9 mM) of 2-amino-1-phenylethanol and 3.55 g (25.9 mM) of methyl 2-(4-nitrophenyl)ethyl ketone in 60 ml of toluene containing 10 mg of *para*-toluenesulfonic acid was heated at reflux for six hours in a flask equipped with a Dean-Stark trap. The water that formed during the reaction was removed via the trap, and then the reaction mixture was cooled to room temperature and concentrated to dryness by evaporation of the solvent under reduced pressure. The solid product that remained was dissolved in 50 ml of tetrahydrofuran and the solution was heated to reflux. A solution of 13.5 ml of 2N borane-methyl sulfide complex in tetrahydrofuran was then added dropwise to the reaction mixture, and the mixture was refluxed for an additional ninety minutes. After cooling the reaction mixture to room temperature, it was diluted by addition of diethyl ether saturated with hydrogen chloride. The precipitate that formed was collected by filtration and crystallized from ethanol and diethyl ether to give 3.29 g of 1-phenyl-2-[1-methyl-3-(4-nitrophenyl)propylamino]ethanol hydrochloride. M.P. 203—213°C.

Analysis calc. for $C_{18}H_{23}ClN_2O_3$

| | | | | |
|---|---|---|---|---|
| Theory: | C, 61.62; | H, 6.61; | N, 7.98; | Cl, 10.11. |
| Found: | C, 61.76; | H, 6.62; | N, 7.76; | Cl, 10.13. |

Example 5B

(R,S)(S,R)-1-Phenyl-2-[1-methyl-3-(4-nitrophenyl)propylamino]ethanol, hydrochloride

Example 5A was repeated exactly, except that the free base obtained from the borane-methyl sulfide reduction was not converted to the hydrochloride salt but, instead, was subjected to multiple recrystallizations from acetonitrile, benzene, isopropyl alcohol (in that order) to give the free base as the racemic (R,S)(S,R)-diastereomer.

This diastereomer was then converted to the hydrochloride by dissolving it in methanol and adding a solution of ethereal hydrogen chloride. The hydrochloride salt thus formed had a melting point of 198 to 202°C.

Example 6

1-(4-Hydroxyphenyl)-2-[1,1-dimethyl-3-phenylpropylamino]ethanol

A solution of 32.6 g (0.2 m) of 1,1-dimethyl-3-phenylpropylamine and 22.9 g (0.1 m) of 2-(4-methoxyphenyl)-2-oxoethyl bromide in 75 ml of tetrahydrofuran was heated at reflux for thirty-six hours. The reaction mixture was then cooled and added to 2 liters of diethyl ether. The precipitate was removed by filtration, and the filtrate was acidified with hydrobromic acid to give 18.15 g of 1-(4-methoxyphenyl)-1-oxo-2-(1,1-dimethyl-3-phenylpropylamino)ethane hydrobromide. M.P. 174—178°C.

The compound thus formed was dissolved in 85 ml of glacial acetic acid containing 35 ml of hydrobromic acid, and the solution was heated at reflux for nine hours. The solution was then cooled and the solvent was removed by evaporation to provide, following crystallization from ethanol and diethyl ether, 7.8 g of 1-(4-hydroxyphenyl)-1-oxo-2-(1,1-dimethyl-3-phenylpropylamino)ethane hydrobromide. M.P. 228—230°C.

Catalytic hydrogenation of 5.0 g of the compound from above in 44 ml of ethanol containing 1.25 g of five percent palladium on carbon afforded, following crystallization from acetone and diethyl ether, 2.3 g of 1-(4-hydroxyphenyl)-2-(1,1-dimethyl-3-phenylpropylamino)ethanol hydrobromide. M.P. 168—170°C.

Analysis calc. for $C_{19}H_{26}BrNO_2$

| | | | |
|---|---|---|---|
| Theory: | C, 60.00; | H, 6.89; | N, 3.68. |
| Found: | C, 60.28; | H, 6.67; | N, 3.62. |

Example 7

1-(3-Hydroxyphenyl)-2-[1,1-dimethyl-3-(4-fluorophenyl)propyl]amino]ethanol

To a stirred solution of 67.2 g (0.22 M) of 2-(3-benzyloxyphenyl)-2-oxoethyl bromide in 200 ml of acetonitrile was added a solution of 54.3 g (0.20 M) of N-benzyl-1,1-dimethyl-3-(4-fluorophenyl)propylamine in 600 ml of acetonitrile containing 42 ml (0.22 M) of diisopropylethylamine. The reaction mixture was stirred for about six hours at reflux, and then was cooled and stored at room temperature for forty-eight hours. The reaction mixture was next diluted by addition of aqueous sodium hydroxide, and the organic solvents were removed by evaporation under reduced pressure. The aqueous alkaline mixture was extracted with ethyl acetate, which was then washed with water, dried, and concentrated to dryness to give an oil. The oil was dissolved in ethanol and diethyl ether and converted to the hydrochloride salt by addition of excess hydrogen chloride. The precipitated solid was collected by filtration and recrystallized from ethyl acetate to give 51.3 g of 1-(3-benzyloxyphenyl)-1-oxo-2-[(N-benzyl)-1,1-dimethyl-3-(4-fluorophenyl)propylamino]ethane hydrochloride. M.P. 137—145°C.

The compound thus prepared was reduced by reaction with 16 g of sodium borohydride in ethanol. Isolation of the product, followed by conversion to the hydrochloride salt and crystallization from diethyl ether afforded 55.0 g of 1-(3-benzyloxyphenyl)-2-[(N-benzyl)-1,1-dimethyl-3-(4-fluorophenyl)propylamino]-ethanol hydrochloride. M.P. 186.5—191°C.

A solution of 10.0 g of the product thus produced in 200 ml of ethanol containing 3.0 g of Raney Nickel was shaken for two hours at 25°C under hydrogen at 44 psi. The reaction mixture was then filtered, and the solvent was removed from the filtrate by evaporation under reduced pressure to give a white solid. The solid was triturated with ethyl acetate and air dried to provide 5.9 g of 1-(3-hydroxyphenyl)-2-[1,1-dimethyl-3-(4-fluorophenyl)propylamino)]ethanolhydrochloride. M.P. 196.5—198.5°C.

Analysis calc. for $C_{19}H_{25}ClFNO_2$

| | | | | |
|---|---|---|---|---|
| Theory: | C, 64.49; | H, 7.12; | N, 3.96; | Cl, 10.02. |
| Found: | C, 64.29; | H, 6.97; | N, 4.06; | Cl, 9.89. |

The following compounds were similarly prepared

Example 8

1-(2-Fluorophenyl)-2-[1,1-dimethyl-3-(4-aminocarbonylphenyl)propylamino]ethanol hydrochloride M.P. 227—230°C.

Example 9

1-(3-Hydroxyphenyl)-2-[1,1-dimethyl-3-(4-hydroxyphenyl)propylamino]ethanol hydrobromide M.P. 161—165°C.

Example 10

1-Phenyl-2-[1-methyl-3-(4-methylsulfonylphenyl)propylamino]ethanol hydrochloride

Methyl 2-(4-methylthiophenyl)ethyl ketone was oxidized by reaction with *m*-chloroperbenzoic acid to give methyl 2-(4-methylsulfonylphenyl)ethyl ketone.

A solution of 6.73 g of 2-amino-1-phenylethanol and 11.10 g of methyl 2-(4-methylsulfonylphenyl)ethyl ketone in 500 ml of toluene containing 200 mg of *p*-toluenesulfonic acid was heated at reflux for twenty-four hours. The reaction mixture was cooled and the solvent was removed by evaporation to give the Schiff base 1-phenyl-2-[1-methyl-3-(4-methylsulfonylphenyl)propylimino]ethanol. The Schiff base thus prepared was reacted with 3.7 g of sodium borohydride in 500 ml of ethanol at 0°C for sixteen hours. The reaction mixture was diluted by addition of 50 ml of acetone and 20 ml of 3N hydrochloric acid. The mixture was concentrated to an oil by evaporation of the solvent. The oil crystallized upon standing at room temperature. Recrystallization of the product from 200 ml of hot ethanol afforded 8.96 g (48% yield) of 1-phenyl-2-[1-methyl-3-(4-methylsulfonylphenyl)propylamino]ethanol hydrochloride. M.P. 164—170°C.

Analysis calc. for $C_{19}H_{26}ClNO_3S$

| | | | | | |
|---|---|---|---|---|---|
| Theory: | C, 59.44; | H, 6.83; | N, 3.65; | Cl, 9.23; | S, 8.35. |
| Found: | C, 59.28; | H, 6.57; | N, 3.70; | Cl, 9.36; | S, 8.11. |

Example 11

Premix for Chickens

| Ingredient | % by weight |
|---|---|
| 1-(4-hydroxyphenyl)-2-[1,1-dimethyl-3-phenylpropylamino]ethanol succinate | 25 |
| Ground Corn | 74 |
| Sodium Chloride | 1 |
| | 100 |

Example 12
Feed Ration for Lambs

| Ingredient | Percent | kg/t | lbs/T |
|---|---|---|---|
| Yellow corn | 61.00 | 610.0 | 1220.0 |
| Corn cobs | 20.00 | 200.0 | 400.0 |
| Alfalfa Meal, dehydrated | 5.40 | 54.0 | 108.0 |
| Soybean oil meal | 8.00 | 80.0 | 160.0 |
| Urea, feed grade | 0.50 | 5.0 | 10.0 |
| Molasses, cane | 3.00 | 30.0 | 60.0 |
| Dicalcium phosphate | 0.43 | 4.3 | 8.6 |
| Salt | 0.30 | 3.0 | 6.0 |
| Calcium carbonate | 0.14 | 1.2 | 2.3 |
| Trace mineral premix[1] | 0.03 | 0.3 | 0.6 |
| Vitamin A + $D_3$ Premix[2] | 0.10 | 1.0 | 2.0 |
| Vitamin E Premix[3] | 0.10 | 1.0 | 2.0 |
| 1-(4-Hydroxyphenyl)-2-(1,1-dimethyl-3-phenylpropylamino)-ethanol | 1.00 | 10.0 | 20.0 |
| | 100.00 | 1000.0 | 2000.0 |

[1] Trace mineral premix contains: 2.5% manganese as manganese oxide, 0.07% iodine as potassium iodide, 0.3% cobalt as cobalt carbonate, 0.5% copper as copper oxide, and 20.0% zinc as zinc sulfate.

[2] Each pound of vitamin A and $D_3$ premix contains 2,000,000 USP units Vitamin A and 225,750 USP units Vitamin $D_3$.

[3] Each pound of Vitamin E premix contains 20,000 IU Vitamin E.

The compounds to be employed in the method of this invention have demonstrated efficacy in animal tests designed to establish beneficial nutritional effects. In one test designed to show lipolytic activity, normal swine, either barrows or gilts, were employed to analyze the effect of compounds on blood glucose, insulin, and non-esterified fatty acids (NEFA).

Test animals were maintained in metabolism crates, and following a period of adaptation, catheters were placed in their femoral veins. Prior to administration of the test compounds, all animals were fasted for a period of forty hours. Such fasting causes an elevation of NEFA's in the blood. On the day of the test, all animals were fed a normal feed ration, and one group of animals were held as controls while another group of animals received a test compound. The test compounds were administered at 200 mcg/kg intravenously, or orally at 1 mg/kg. Blood samples were taken from each animal immediately prior to treatment and then at one hour intervals for a period of six hours following treatment. The blood plasma was analyzed for glucose, insulin and NEFA content.

When the fasted pigs were fed the normal feed ration without a test compound, the NEFA levels in the blood drop dramatically and remained low. A β-phenethanolamine as defined herein caused either an elevation in NEFA's, or inhibited the drop in NEFA's. Blood levels of glucose and insulin were also elevated with the β-phenethanolamines.

The following Table presents the lipolytic activity of several preferred β-phenethanolamines when evaluated according to the test described above. The results are averages of several tests.

TABLE I

Lipolytic Activity (increase in NEFA's)

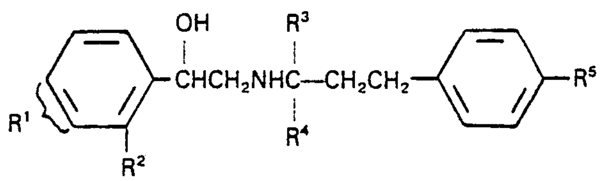

| $R^1$ | $R^2$ | $R^3$ | $R^4$ | $R^5$ | % increase in NEFA's over control | % Increase in glucose over control |
|---|---|---|---|---|---|---|
| H | H | H | $CH_3$ | $SO_2CH_3$ | 131 | 9 |
| *p*-OH | H | $CH_3$ | $CH_3$ | H | 445 | 48 |
| *m*-OH | H | $CH_3$ | $CH_3$ | H | 71 | 31 |
| *m*-OH | H | $CH_3$ | $CH_3$ | F | 28 | 72 |
| *p*-OH | H | $CH_3$ | $CH_3$ | OH | 141 | 35 |
| *m*-OH | H | $CH_3$ | $CH_3$ | OH | 68 | 40 |
| H | H | H | H | $NO_2$ | 199 | 7 |
| *p*-$OCH_3$ | H | $CH_3$ | $CH_3$ | H | 84 | 25 |
| *p*-$OCH_3$ | H | $CH_3$ | $CH_3$ | OH | 249 | 5 |
| H | H | H | $CH_3$ | $NO_2$ | 1458 | 27 |

A ten day *in vivo* study was employed to determine the effect of β-phenethanolamines on feed efficiency and rate of growth. In these studies, barrows and gilts weighing approximately 60 kg were maintained in individual pens. Each treatment was replicated six times in randomly assigned animals, with each test animal forming an experimental unit. A group of control animals received a normal swine grower feed ration comprising the following ingredients:

| Ingredient | % by weight |
|---|---|
| Ground yellow corn | 76.70 |
| Soybean oil meal | 19.35 |
| Calcium carbonate | 1.20 |
| Dicalcium phosphate | 1.20 |
| Salt | 0.50 |
| Trace mineral premix | 0.10 |
| Swine Vitamin premix | 0.65 |
| Vitamin A premix, 3M USP units/lb. | 0.05 |
| Methionine Hydroxy analogue, 93% | 0.20 |
| Selenium premix | .05 |
| | 100.00 |

The test animals received the same feed ration plus the test compound. All animals received feed and water *ad libitum.* All animals were weighed on day 1 and again on day 10, and feed consumption was measured by recording all feed issued and any remaining feed on day 10. The results of two series of this 10 day test for several β-phenethanolamines are given in Table II. In the Table, the column labelled "ADG" is the average daily weight gain in kilograms and pounds; "ADF" is the average daily feed consumption (in kilograms and pounds) by the test animals; and F/G is the feed efficiency calculated as ADF divided by ADG.

TABLE II

Growth Promotion and Feed Efficiency

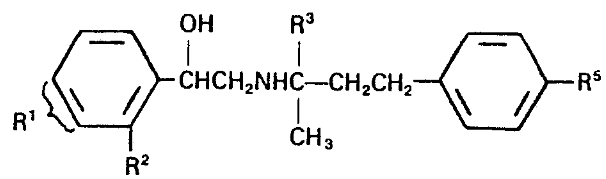

| | $R^1$ | $R^2$ | $R^3$ | $R^5$ | dose ppm | ADG | | ADF | | F/G |
|---|---|---|---|---|---|---|---|---|---|---|
| | | | | | | kg | (lb) | kg | (lb) | |
| Experiment I | Control | | | | | 0.73 | (1.60) | 2.13 | (4.7) | 2.98 |
| | H | H | H | $NO_2$ | 20 | 0.81 | (1.78) | 1.91 | (4.22) | 2.37 |
| Experiment II | Control | | | | | 0.61 | (1.34) | 1.89 | (4.16) | 3.22 |
| | *p*-OH | H | $CH_3$ | H | 20 | 0.73 | (1.60) | 1.93 | (4.26) | 2.66 |
| | *m*-OH | H | $CH_3$ | F | 20 | 0.69 | (1.52) | 2.07 | (4.57) | 3.01 |

The β-phenethanolamines to be employed in the method of this invention can be administered in combination with other compounds known to have a beneficial effect upon animals. Typical compounds to be co-administered with the β-phenethanolamines include antibiotics, for example any of the tetracyclines, tylosin, penicillins, cephalosporins, polyether antibiotics, glycopeptides, orthosomycins and related compounds commonly administered to swine, poultry, ruminants and the like. Such combinations will comprise the respective components in a ratio of about 1 to about 2 parts by weight of β-phenethanolamine and about 1 to about 10 parts by weight of the partner component.

A total of 180 crossbred barrows and gilts, averaging approximately 134 pounds starting weight, were used to determine the effect of oral administration of Examples 5A, 6 and 7 at 5 or 20 ppm on growth performance and carcass characteristics of finishing pigs. The pigs were fed a 16% crude protein corn-soy diet during the experiment and were housed in an enclosed commercial type swine building with concrete slatted floors. The effect of treatments on average daily gain (ADG), average daily feed (ADF) and feed/gain are shown in Table III. The treatment effects on the carcass composition of the pigs are presented in Table IV.

TABLE III

| Compound | | Number Animal | No. Pens | Initial Wt. kg | Initial Wt. lb | ADG kg | ADG lb | ADF kg | ADF lb | Feed/ Gain |
|---|---|---|---|---|---|---|---|---|---|---|
| Control | | 19 | 4 | 61 | (134) | 0.68 | (1.51) | 2.40 | (5.30) | 3.50 |
| Example 6 | 5 ppm | 19 | 4 | 60 | (133) | 0.74 | (1.64) | 2.51 | (5.54) | 3.38 |
| Example 6 | 20 ppm | 20 | 4 | 61 | (135) | 0.75 | (1.66) | 2.42 | (5.33) | 3.21 |
| Example 7 | 5 ppm | 20 | 4 | 61 | (134) | 0.71 | (1.57) | 2.49 | (5.49) | 3.49 |
| Example 7 | 20 ppm | 18 | 4 | 61 | (135) | 0.73 | (1.61) | 2.53 | (5.57) | 3.47 |
| Example 5A | 5 ppm | 19 | 4 | 61 | (134) | 0.73 | (1.60) | 2.41 | (5.32) | 3.31 |
| Example 5A | 20 ppm | 18 | 4 | 61 | (134) | 0.76 | (1.68) | 2.47 | (5.45) | 3.24 |

Animals were slaughtered on a weight constant basis at approximately 103.5 kilograms (230 pounds).

Animals were on test an average of 60 days. The control feed and all treated feeds contained Tylosin at 44 ppm (40 g/t).

TABLE IV

| Treatment Description | Ave. Live wt. kg/lb | Ave. Ave. Yield % | Carc. Leng. cm/in | Leaf-Fat Wt. kg/lb | Ave. Ave. B.F. cm/in | Loin Fat Depth cm/in | Eye Area sq. cm./sq. in. | Est. % Musc. | Est. kg/lb Musc. |
|---|---|---|---|---|---|---|---|---|---|
| Control | 103 (228) | 70.0 | 82.3 (32.4) | 1.22 (2.70) | 2.79 (1.10) | 2.29 (0.90) | 32.58 (5.05) | 50.9 | 37.7 (83.1) |
| Example 6 5 ppm | 105 (232) | 70.8 | 81.8 (32.2) | 1.37 (3.01) | 2.77 (1.09) | 2.41 (0.95) | 34.71 (5.38) | 51.5 | 39.2 (86.4) |
| Example 6 20 ppm | 105 (231) | 71.4 | 81.3 (32.0) | 1.16 (2.55) | 2.51 (0.99) | 2.06 (0.81) | 36.77 (5.70) | 53.4 | 40.8 (89.9) |
| Example 7 5 ppm | 105 (232) | 70.9 | 82.6 (32.5) | 1.30 (2.87) | 2.79 (1.10) | 2.44 (0.96) | 33.16 (5.14) | 50.7 | 38.7 (85.3) |
| Example 7 20 ppm | 105 (232) | 71.4 | 82.6 (32.5) | 1.32 (2.91) | 3.23 (1.27) | 2.41 (0.95) | 34.84 (5.40) | 51.5 | 39.5 (87.1) |
| Example 5A 5 ppm | 104 (230) | 71.2 | 82.3 (32.4) | 1.21 (2.66) | 2.82 (1.11) | 2.24 (0.88) | 33.61 (5.21) | 51.6 | 39.1 (86.2) |
| Example 5A 20 ppm | 104 (230) | 70.9 | 81.5 (32.1) | 1.15 (2.54) | 2.74 (1.08) | 2.06 (0.81) | 36.19 (5.61) | 53.2 | 40.1 (88.4) |

KEY: Ave. = Average; B.F. = Back Fat; Est. = Estimated; Musc. = Muscle Fat.

Additional studies have been carried out to demonstrate the anabolic effect of β-phenethanolamines in swine. The effect of the compounds on nitrogen retention in finishing barrows was determined. Nitrogen retention is the difference between nitrogen intake and nitrogen excreted. Increased nitrogen retention is known to be associated with increased anabolic activity, resulting in improved carcass muscling and leanness.

**Claims**

1. A method for promoting the growth, improving the efficiency of feed utilisation or improving the leanness of a domesticated animal, which comprises administering to said animal a compound of the formula (I):

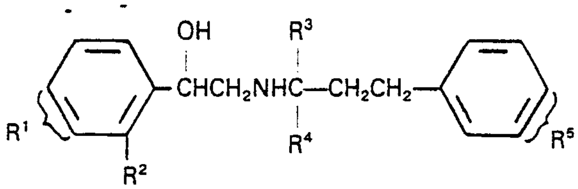

(I)

wherein:
$R^1$ is hydrogen, hydroxy, or methoxy;
$R^2$ is hydrogen or fluoro;
$R^3$ is hydrogen or $C_1$—$C_2$ alkyl;
$R^4$ is methyl;
$R^5$ is hydrogen, fluoro, nitro, hydroxy or $SO_2CH_3$; provided that $R^1$ is hydrogen only when $R^5$ is nitro or $SO_2CH_3$ and provided that when $R^1$ and $R^5$ are both *para* hydroxy and $R^2$ is hydrogen, $R^3$ is $C_1$—$C_2$ alkyl; or an acid addition salt thereof.

2. A method as claimed in claim 1 wherein in formula (I) $R^1$ is hydroxy and $R^2$ is hydrogen.

3. A method as claimed in claim 1 or 2 wherein in formula (I) $R^5$ is nitro, hydroxy or hydrogen.

4. A method as claimed in claim 1, 2 or 3 wherein in formula (I) $R^1$ and/or $R^5$ are *para*-substituents.

5. An animal feed premix comprising a β-phenethanolamine of the formula (I):

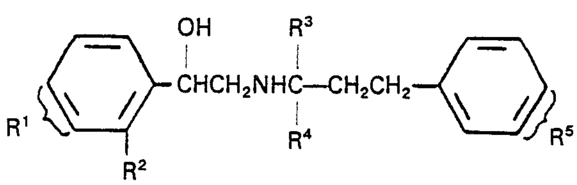

(I)

wherein:
$R^1$ is hydrogen, hydroxy, or methoxy;
$R^2$ is hydrogen or fluoro;
$R^3$ is hydrogen or $C_1$—$C_2$ alkyl;
$R^4$ is methyl;
$R^5$ is hydrogen, fluoro, nitro, hydroxy or $SO_2CH_3$; provided that $R^1$ is hydrogen only when $R^4$ is $SO_2CH_3$ or nitro and provided that when $R^1$ and $R^5$ are both *para* hydroxy and $R^2$ is hydrogen, $R^3$ is $C_1$—$C_2$ alkyl; or an acid addition salt thereof, associated with a suitable carrier therefor.

6. An animal feed premix as claimed in claim 5, wherein the compound of formula (I) is as defined in any one of claims 2 to 4.

7. 1-Phenyl-2-[1-methyl-3-(4-nitrophenyl)-propylamino]ethanol, or an acid-addition salt thereof.

8. 1-Phenyl-2-[1-methyl-3-(4-nitrophenyl)-propylamino]ethanol, hydrochloride.

9. The (R,S) (S,R)-diastereomer of the compound claimed in claim 7 or 8.

10. 1-(4-Hydroxyphenyl)-2-[1,1-dimethyl-3-phenylpropylamino]ethanol, or an acid-addition salt thereof.

11. 1-(3-Hydroxyphenyl)-2-[1,1-dimethyl-3-(4-fluorophenyl)propylamino]ethanol, or an acid-addition salt thereof.

**Patentansprüche**

1. Verfahren zur Förderung des Wachstums, Verbesserung der Effizienz der Futterausnutzung oder Verbesserung der Magersucht eines domestizierten Tieres, dadurch gekennzeichnet, daß einem solchen Tier eine Verbindung der Formel (I)

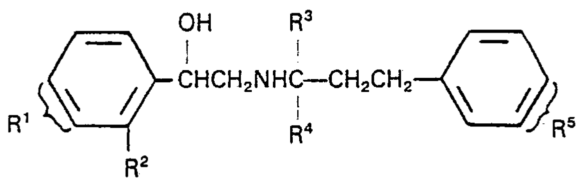

(I)

worin

$R^1$ Wasserstoff, Hydroxy oder Methoxy ist,

$R^2$ Wasserstoff oder Fluor bedeutet,

$R^3$ Wasserstoff oder $C_1$—$C_2$-Alkyl ist,

$R^4$ Methyl darstellt und

$R^5$ Wasserstoff, Fluor, Nitro, Hydroxy oder $SO_2CH_3$ ist,

mit der Maßgabe, daß $R^1$ nur dann Wasserstoff ist, wenn $R^5$ Nitro oder $SO_2CH_3$ bedeutet, und daß $R^3$ für $C_1$—$C_2$-Alkyl steht, falls $R^1$ und $R^5$ jeweils p-Hydroxy sind und $R^2$ Wasserstoff ist, oder ein Säureadditionssalz hiervon verabreicht wird.

2. Verfahren nach Anspruch 1, dadurch gekennzeichnet, daß $R^1$ Hydroxy ist und $R^2$ Wasserstoff bedeutet.

3. Verfahren nach Anspruch 1 oder 2, dadurch gekennzeichnet, daß $R^5$ Nitro, Hydroxy oder Wasserstoff ist.

4. Verfahren nach Anspruch 1, 2 oder 3, dadurch gekennzeichnet, daß $R^1$ und/oder $R^5$ p-Substituenten sind.

5. Futtervormischung für Tiere, dadurch gekennzeichnet, daß sie ein β-Phenethanolamin der Formel (I)

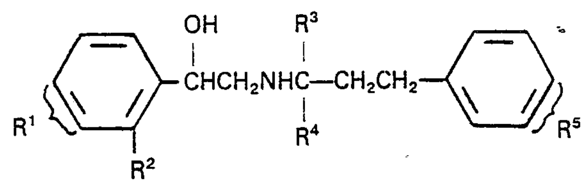

(I)

worin

$R^1$ Wasserstoff, Hydroxy oder Methoxy ist,

$R^2$ Wasserstoff oder Fluor bedeutet,

$R^3$ Wasserstoff oder $C_1$—$C_2$-Alkyl ist,

$R^4$ Methyl darstellt und

$R^5$ Wasserstoff, Fluor, Nitro, Hydroxy oder $SO_2CH_3$ ist,

mit der Maßgabe, daß $R^1$ nur dann Wasserstoff ist, wenn $R^5$ Nitro oder $SO_2CH_3$ bedeutet, und daß $R^3$ für $C_1$—$C_2$-Alkyl steht, falls $R^1$ und $R^5$ jeweils p-Hydroxy sind und $R^2$ Wasserstoff ist, oder ein Säureadditionssalz hiervon in Kombination mit einem geeigneten Träger hierfür enthält.

6. Futtervormischung für Tiere nach Anspruch 5, dadurch gekennzeichnet, daß sie als Verbindung der Formel (I) irgendeine der Verbindungen der Ansprüche 2 bis 4 enthält.

7. 1-Phenyl-2-[1-methyl-3-(4-nitrophenyl)propylamino]ethanol oder ein Säureadditionssalz hiervon.

8. 1-Phenyl-2-[1-methyl-3-(4-nitrophenyl)propylamino]ethanol-hydrochlorid.

9. (R,S) (S,R)-Diastereoisomeres der Verbindung nach Anspruch 7 oder 8.

10. 1-(4-Hydroxyphenyl)-2-[1,1-dimethyl-3-phenylpropylamino]ethanol oder ein Säureadditionssalz hiervon.

11. 1-(3-Hydroxyphenyl)-2-[1,1-dimethyl-3-(4-fluorpłphenyl)opylamino]ethanol oder ein Säureadditionssalz hiervon.

**Revendications**

1. Procédé en vue d'activer la croissance, d'améliorer l'efficacité de l'utilisation de la nourriture ou la qualité de viande maigre d'un animal domestiqué, caractérisé en ce qu'il consiste à administrer, à cet animal, un composé répondant à la formule (I):

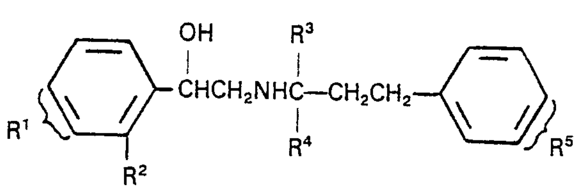

(I)

dans laquelle:

$R^1$ représente un atome d'hydrogène, un groupe hydroxyle ou un groupe methoxy;

$R^2$ représente un atome d'hydrogène ou un atome de fluor;

$R^3$ représente un atome d'hydrogène ou un groupe alkyle en $C_1$—$C_2$;

$R^4$ représente un groupe méthyle;

$R^5$ représente un atome d'hydrogène, un atome de fluor, un groupe nitro, un groupe hydroxyle ou $SO_2CH_3$; avec cette réserve que $R^1$ représente un atome d'hydrogène uniquement lorsque $R^5$ représente un groupe nitro ou $SO_2CH_3$ et que, lorsque $R^1$ et $R^5$ représentent tous deux un groupe hydroxyle en position para et $R^2$ représente un atome d'hydrogène, $R^3$ représente un groupe alkyle en $C_1$—$C_2$; ou encore un sel d'addition d'acide de ce composé.

2. Procédé selon la revendication 1, caractérisé en ce que, dans la formule (I), $R^1$ représente un groupe hydroxyle et $R^2$ représente un atome d'hydrogène.

3. Procédé selon la revendication 1 ou 2, caractérisé en ce que, dans la formule (I), $R^5$ représente un groupe nitro, un groupe hydroxyle ou un atome d'hydrogène.

4. Procédé selon une des revendications 1 à 3, caractérisé en ce que, dans la formule (I), $R^1$ et/ou $R^5$ sont des substituants en position para.

5. Prémélange de nourriture pour animaux, caractérisé en ce qu'il contient une β-phénéthanolamine répondant à la formule (I):

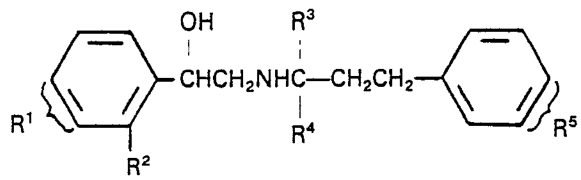

(I)

dans laquelle:

$R^1$ représente un atome d'hydrogène, un groupe hydroxyle ou un groupe méthoxy;

$R^2$ représente un atome d'hydrogène ou un atome de fluor;

$R^3$ représente un atome d'hydrogène ou un groupe alkyle en $C_1$—$C_2$;

$R^4$ représente un groupe méthyle;

$R^5$ représente un atome d'hydrogène, un atome de fluor, un groupe nitro, un groupe hydroxyle ou $SO_2CH_3$; avec cette réserve que $R^4$ représente un atome d'hydrogène uniquement lorsque $R^4$ représente $SO_2CH_3$ ou un groupe nitro et que, lorsque $R^1$ et $R^5$ représentent tous deux un groupe hydroxyle en position para et $R^2$ représente un atome d'hydrogène, $R^3$ représente un groupe alkyle en $C_1$—$C_2$; ou encore un sel d'addition d'acide de ce composé, en association avec un support approprié.

6. Prémélange de nourriture pour animaux selon la revendication 5, caractérisé en ce que le composé de formule (I) est un composé semblable à celui défini dans l'une quelconque des revendications 2 à 4.

7. 1-phényl-2-[1-méthyl-3-(4-nitrophényl)-propylamino]éthanol ou un de ses sels d'addition d'acide.

8. Chlorhydrate de 1-phényl-2-[1-méthyl-3-(4-nitrophényl)-propylamino]éthanol.

9. Le (R,S)(S,R)-diastéréoisomère du composé selon la revendication 7 ou 8.

10. 1-(4-hydroxyphényl)-2-[1,1-diméthyl-3-phénylpropylamino]éthanol ou un de ses sels d'addition d'acide.

11. 1-(3-hydroxyphényl)-2-[1,1-diméthyl-3-(4-fluorophényl)-propylamino]éthanol ou un de ses sels d'addition d'acide.